(19) Europäisches Patentamt
European Patent Office
Office européen des brevets

(11) Veröffentlichungsnummer: **0 010 759**
**B1**

(12) # EUROPÄISCHE PATENTSCHRIFT

(45) Veröffentlichungstag der Patentschrift: **30.07.86**

(21) Anmeldenummer: **79104238.5**

(22) Anmeldetag: **31.10.79**

(51) Int. Cl.[4]: **C 07 D 471/04,** C 07 D 491/147, C 07 F 9/65, A 61 K 31/505, A 61 K 31/675 // (C07D471/04, 239:00, 221:00),(C07D491/147, 317:00, 239:00, 221:00),(C07D491/147, 319:00, 239:00, 221:00)

(54) **Pyrimido-(6,1-a)-isochinolin-4-one, neue bei ihrer Herstellung verwendete Zwischenprodukte, Verfahren zu ihrer Herstellung und ihre Verwendung als Arzneimittel.**

(30) Priorität: **03.11.78 DE 2847693**

(43) Veröffentlichungstag der Anmeldung: **14.05.80 Patentblatt 80/10**

(45) Bekanntmachung des Hinweises auf die Patenterteilung: **30.07.86 Patentblatt 86/31**

(84) Benannte Vertragsstaaten: **AT BE CH DE FR GB IT NL SE**

(56) Entgegenhaltungen:
**BE-A- 862 785**

**CHEMICAL ABSTRACTS, Band 87, Nr. 11, Sept. 12, 1977, Seite 577, Zus. 84789w COLUMBUS, OHIO (US) J. KOBOR: "Study on isoquinoline compounds. Study on the reduction of 1-(ethoxycarbonylmethylene)- 6,7-dimethoxy-1,2,3,4-tetrahydro-isoquinoline phenyl isocyanate"**

(73) Patentinhaber: **HOECHST AKTIENGESELLSCHAFT**
**Postfach 80 03 20**
**D-6230 Frankfurt am Main 80 (DE)**

(72) Erfinder: **Lal, Bansi, Dr.**
**Advani Apartments 30**
**Mulund (West), Bombay (IN)**
Erfinder: **D'Sa, Adolf, Dr.**
**Laxmi Apartments 17 Station Road**
**Vikhroli (West), Bombay (IN)**
Erfinder: **Dornauer, Horst, Dr.**
**Chellaram House Carmichael Road**
**Bombay (IN)**
Erfinder: **DE Souza, Noel John, Dr.**
**Avanti 702 Central Avenue**
**Santa Cruz, Bombay (IN)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 010 759 B1

**Beschreibung**

Die vorliegende Erfindung betrifft Pyrimido-(6,1-a)-isochinolin-4-onderivate, neue bei ihrer Herstellung verwendete Zwischenprodukte und ein neues Verfahren zur Herstellung der Pyrimido-(6,1-a)-isochinolin-4-one.

Die vorliegende Erfindung betrifft Pyrimido-(6,1-a)-isochinolin-4-one der Formel I

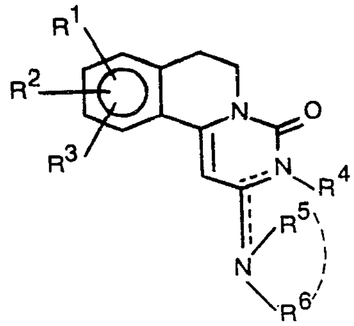

in welcher

$R^1$ und $R^2$ unabhängig voneinander Wasserstoff, Alkoxy mit 1 bis 3 Kohlenstoffatomen, Hydroxy-, Methylendioxy oder Äthylendioxy bedeuten,

$R^3$ Wasserstoff oder Alkoxy mit 1 bis 3 Kohlenstoffatomen bedeutet,

$R^4$ Wasserstoff oder Alkyl mit 1 bis 4 Kohlenstoffatomen, oder Di($C_{1-4}$-Alkyl)-amino-$C_{1-4}$-alkyl) bedeutet und $R^4$ für ein Elektronenpaar steht, falls $R^5$ für einen der unten angegebenen Reste steht,

$R^5$ Wasserstoff, Alkyl mit 1 bis 6 Kohlenstoffatomen, Hydroxyalkyl mit 1 bis 6 Kohlenstoffatomen, Alkanoyl mit 1 bis 6 Kohlenstoffatomen oder Benzoyl, wobei der Phenylrest 1—3-fach substituiert sein kann mit Halogen, Trifluormethyl, $C_1$—$C_3$-Alkyl, $C_1$—$C_3$-Alkoxy, Amino, Hydroxy, O-Alkali, bedeutet und $R^5$ für ein Elektronenpaar steht, falls $R^4$ für einen der oben angegebenen Reste steht,

$R^6$ ,Wasserstoff, Hydroxyl, Amino, $C_1$—$C_3$-Alkylamino, Di-$C_1$—$C_3$-Alkylamino, $C_1$—$C_6$-Alkyl, das substituiert sein kann mit Halogen, Hydroxy, $C_1$—$C_3$-Alkoxy, Amino oder Di-($C_1$—$C_4$-alkyl)-amino, Cyclohexyl Phen-$C_1$—$C_2$-alkyl, Phenyl, welches 1—3-fach substituiert sein kann mit Halogen. Trifluormethyl, $C_1$—$C_3$-Alkyl, $C_1$—$C_3$-Alkoxy, Amino, Hydroxy oder O-Alkali, oder $R^5$ und $R^6$ zusammen mit dem N-Atom, an das sie gebunden sind, den Pyrrolidino- oder Morpholino-Rest oder den Piperazino-Rest, welche, am N durch $C_1$-$C_3$-Alkyl, $C_2$—$C_4$-Alkoxycarbonyl oder Phenyl, welches 1—3 fach substituiert sein kann mit Halogen, Trifluormethyl, $C_1$—$C_3$-Alkyl, $C_1$—$C_3$-Alkoxy, Amino, Hydroxy oder O-Alkali, substituiert sein können, sowie deren Säureadditionssalze und quaternären Ammoniumsalze.

Gegenstand der vorliegenden Erfindung sind ferner Pyrimido-(6,1-a)-isochinolin-4-one der Formel II

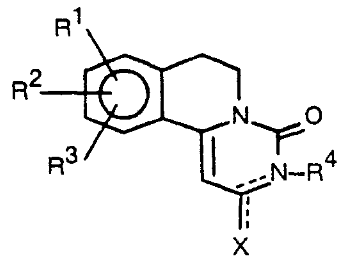

II

worin $R^1$, $R^2$, $R^3$ die zur Formel I genannte Bedeutung haben, zusätzlich jedoch auch Halogen bedeuten können, $R^4$ die zur Formel I genannte Bedeutung hat, in welchem Falle X Schwefel bedeutet, oder $R^4$ ein Elektronenpaar darstellt, in welchem Falle X ein Halogenatom bedeutet.

Die Verbindungen der Formel II finden als Zwischenprodukte bei der Herstellung der Verbindungen der Formel I Verwendung.

Falls wenigstens einer der beiden Reste $R^5$ und $R^6$ für ein Wasserstoffatom steht, schließt die obige Definition der Pyrimido-(6,1-a)-isochinolin-4-on-derivate der Formel I auch die der folgenden Formel entsprechenden, entweder durch vollständige Isomerisierung der Verbindungen der Formel Ia erhaltenen oder mit den Verbindungen der Formel Ia im Gleichgewicht stehenden Isomeren Ib ein.

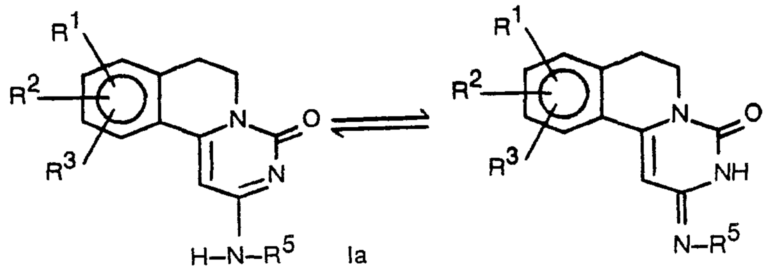

Ia

Ib

Die Definition der Pyrimido-(6,1-a)-isochinolin-4-on-derivate umfaßt auch das Ic-Isomer der folgenden Formel, worin $R^1$, $R^2$, $R^3$, $R^4$ und $R^5$ die obige Bedeutung haben.

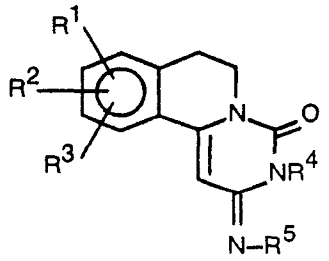

Ic

Falls $R^1$, $R^2$ oder $R^3$ Halogen bedeuten, ist Chlor bevorzugt. Geeignete Alkylreste für $R^4$ sind z.B. Methyl, Äthyl, n-Propyl, Isopropyl, Butyl, Isobutyl, sec.-Butyl oder tert.-Butyl.

Als Alkylamino- oder Dialkylaminoreste für $R^5$ oder $R^6$ eignen sich besonders solche mit Alkylgruppen mit höchstens 3 Kohlenstoffatomen, z.B. Methylamino- oder Dimethylaminogruppen.

Als Alkylreste für $R^5$ oder $R^6$ eignen sich solche mit höchstens 6 Kohlenstoffatomen, wie z.B. Methyl, Äthyl, n-Propyl, Isopropyl, Butyl, Isobutyl, sec.-Butyl oder tert.-Butyl.

Als ein substituierter Alkylrest für $R^5$ oder $R^6$ kann ein Rest mit bis zu 6 Kohlenstoffatomen eingesetzt werden, der mit einer oder zwei Hydroxyl- oder Alkoxygruppen substituiert sein kann, wobei die Alkoxygruppen höchstens je 3 Kohlenstoffatome besitzen, weiterhin Halogen, z.B. Chlor, Amino oder Dialkylamino, wobei die Alkylgruppen höchstens 4 Kohlenstoffatome besitzen.

Beispiele für Phenylalkylreste für $R^5$ oder $R^6$ sind solche mit höchstens 8 Kohlenstoffatomen, in welchen der Phenylrest ein- oder mehrfach, insbesondere ein-, zwei- oder dreifach, mit den oben für $R^1$ angegebenen Substituenten substituiert sein kann.

Geeignete Phenylreste für $R^5$ oder $R^6$ sind beispielsweise gegebenenfalls ein- oder mehrfach, insbesondere ein-, zwei oder dreifach, durch Halogenatome, z.B. Fluor-, Chlor- oder Bromatome, Alkyl- oder Alkoxygruppen mit höchstens 3 Kohlenstoffatomen, z.B. Methyl, Äthyl, Methoxy und Äthoxygruppen, Trifluoromethylgruppen, Amino oder Hydroxygruppen substituierte Phenylreste, wobei die Wasserstoffatome in den Hydroxygruppen durch ein Alkali, z.B. Natrium, ersetzt sein können.

Geeignete Reste von stickstoffhaltigen Heterozyklen sind beispielsweise der Pyrrolidino-, Morpholino- oder Piperazino- Rst, die durch $C_1$—$C_3$-Alkyl, $C_2$—$C_4$-Alkoxycarbonyl, gegebenenfalls wie oben angegeben substituiertes Phenyl substituiert sein können.

Beispiele von geeigneten Acylresten für $R^5$ oder $R^6$ sind geradkettige oder verzweigte Alkanoylreste mit 1 bis 6 Kohlenstoffatomen, wie z.B. Acetyl, oder Benzoyl, wobei der Phenylrest ein- oder mehrfach mit den oben für $R^5$ und $R^6$ falls diese einer Phenylrest bedeuten, aufgeführten Substituenten substituiert sein kann.

Als Salze der Pyrimido-(6,1-a)-isochinolin-4-on-derivate der Formel I seien beispielsweise solche von anorganischen oder organischen Säuren erwähnt, z.B. Hydrochloride, Hydrobromide, Sulfate, Phosphate, Acetate, Oxalate, Tartrate, Citrate, Maleate oder Fumarate.

Geeignete quaternäre Ammoniumsalze der Pyrimido-(6,1-a)-isochinolin-4-on-derivate der Formel I sind z.B. die von Alkylhalogeniden abgeleiteten Salze wie Methyljodide.

Als bevorzugte Substituenten gelten folgende: für $R^1$ und $R^2$ $C_{1-3}$-Alkoxy, für $R^3$ Wasserstoff, für $R^5$ $C_{1-6}$-Alkyl oder gegebenenfalls mit Substituenten der oben angegebenen Art ein- bis dreifach substituiertes Phenyl, für $R^6$ Wasserstoff, für $R^4$ Wasserstoff, wenn X Sauerstoff bedeutet und ein Elektronenpaar, wenn X Chlor bedeutet.

Besonders bevorzugte Verbindungen der Formel I sind:

9,10-Dimethoxy-2-tert.-butylamino-6,7-dihydro-4H-pyrimido-(6,1-a)-isochinolin-4-on-hydrochlorid,
9,10-Dimethoxy-2-sec.-butylamino-6,7-dihydro-4H-pyrimido-(6,1-a)-isochinolin-4-on-hydrochlorid,
9,10-Dimethoxy-2-(2,6-dimethylanilino)-6,7-dihydro-4H-pyrimido-(6,1-a)-isochinolin-4-on,
9,10-Dimethoxy-2-(2,4-dimethylanilino)-6,7-dihydro-4H-pyrimido-(6,1-a)-isochinolin-4-on,
9,10-Dimethoxy-2-(2-chloranilino)-6,7-dihydro-4H-pyrimido-(6,1-a)-isochinolin-4-on-hydrochloridmonohydrat und
9,10-Dimethoxy-2-(2,4,6-trimethylanilino)-6,7-dihydro-4H-pyrimido-(6,1-a)-isochinolin-4-on-hydrochlorid-dihydrat.

Besonders bevorzugte Verbindungen der Formel II sind:

9,10-Dimethoxy-3,4,6,7-tetrahydro-2H-pyrimido-(6,1-a)-isochinolin-2,4-dion,
2-Chlor-9,10-dimethoxy-6,7-dihydro-4H-pyrimido-(6,1-a)-isochinolin-4-on,
2-Chlor-9,10-methylendioxy-6,7-dihydro-4H-pyrimido-(6,1-a)-isochinolin-4-on.

Einige Pyrimido-(6,1-a)-isochinolin-4-on-derivate der Formel II

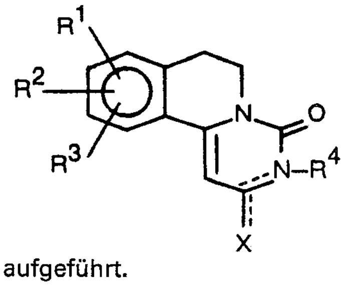

II

sind in nachfolgender Tabelle I aufgeführt.

TABELLE I

| $R^1$ | $R^2$ | $R^3$ | $R^4$ | X | Schmelzpunkt (°C) |
|---|---|---|---|---|---|
| H | H | H | H | O | 308—309 |
| 9—$OCH_3$ | 10—$OCH_3$ | H | H | O | 323—325 |
| H | 10—Cl | H | — | O | 250 |
| H | H | H | — | Cl | 179—180 |
| 9—$OCH_3$ | 10—$OCH_3$ | H | — | Cl | 235—236 |
| H | 10—Cl | H | — | Cl | 250 |
| 9,10(—$OCH_2O$—) | | H | — | Cl | 245—247 |

Die vorliegende Erfindung betrifft weiterhin neue Barbitursäurederivate der Formel III zur Herstellung der Pyrimido-(6,1-a)-isochinolin-4-one der Formel II.

Nachfolgend sind insbesondere zwei neue Barbitursäurederivate nämlich die 1-(3,4-Dimethoxyphenäthyl)-barbitursäure und die 1-(3,4-Methylendioxyphenäthyl)-barbitursäure, mit den entsprechenden Schmelzpunkten aufgeführt:

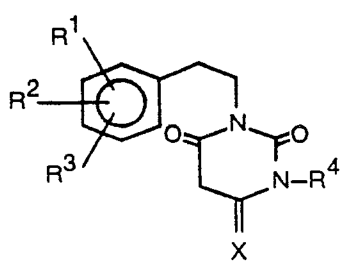

III

TABELLE II

| $R^1$ | $R^2$ | $R^3$ | $R^4$ | X | Schmelzpunkt (°C) |
|---|---|---|---|---|---|
| 3—$OCH_3$ | 4—$(OCH_3)_2$ | H | H | O | 185—187 |
| 3,4(—$OCH_2O$—) | | H | H | O | 222—224 |

Gegenstand der vorliegenden Erfindung ist weiterhin ein Verfahren zur Herstellung der Verbindungen der Formel I, das dadurch gekennzeichnet ist, daß
a) ein Barbitursäurederivat der Formel III

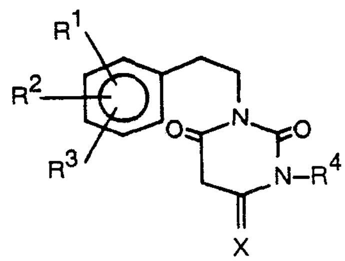

III

worin $R^1$, $R^2$, $R^3$ und $R^4$ die zur Formel I genannten Bedeutungen haben und X Sauerstoff oder Schwefel bedeutet, zyklisiert wird in Gegenwart eines wasserabspaltenden Mittels, z.B. in Gegenwart einer Säure wie z.B. Polyphosphorsäure, Phosphorpentoxid oder Schwefelsäure zu einer Verbindungen der Formel II, worin $R^1$, $R^2$, $R^3$, $R^4$ und X die oben genannten Bedeutungen haben, oder in Gegenwart eines Säurehalogenids wie z.B. Phosphoroxychlorid, Thionylchlorid oder eines entsprechenden anorganischen Halogenids, wie z.B. Phosphorpentachlorid zu einer Verbindung der Formel II, worin $R^1$, $R^2$, $R^3$ und $R^4$ die zur Formel I genannten Bedeutungen haben und X ein Halogenatom, insbesondere Chlor bedeutet, und
b) die erhaltene Verbindung der Formel II mit einer Verbindung der Formel

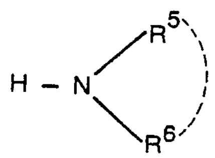

worin $R^5$ und $R^6$ die zur Formel I genannten Bedeutungen haben, in Gegenwart einer Base umgesetzt wird, wonach das Produkt in Form der freien Base mit einer Säure zum Salz umgesetzt werden kann.

Falls für den Verfahrensschritt eine Säure wie z.B. Polyphosphorsäure verwendet wird, kann die Reaktion durch Erhitzen der Reaktionsteilnehmer auf 80—150°C beschleunigt oder vervollständigt werden. Falls ein Säurehalogenid oder ein anorganisches Halogenid verwendet werden, können die Reaktionen in Gegenwart eines Lösungsmittels wie z.B. Äther, z.B. Dioxan oder Tetrahydrofuran, aliphatische halogenierte Kohlenwasserstoffe, z.B. Chloroform oder aromatische Kohlenwasserstoffe wie z.B. Benzol oder Toluol, durchgeführt werden, wobei durch Erhitzen bis zum Siedepunkt des Lösungsmittels eine Beschleunigung erreicht wird.

Beim Verfahrensschritt b) kann es sich bei der Base, in deren Gegenwart die Umsetzung erfolgt, um die Verbindung der Formel

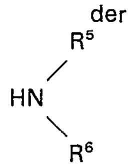

selbst handeln, die im Überschuß über die für die Reaktion benötigte Menge zugesetzt werden kann, oder um ein Alkalihydrid, z.B. Natriumhydrid, oder ein tertiäres Amin, z.B. Triäthylamin, oder einen Säurefänger, z.B. Diazabicyclononen. Die Reaktion kann in Gegenwart polarer Lösungsmittel, z.B. Dimethylformamid, Dimethylsulfoxid, aliphatische halogenierte Kohlenwasserstoffe, z.B. Chloroform, oder Alkanole, z.B. Butanol, oder in Gegenwart aprotischer Lösungsmittel, z.B. hochsiedender Äther, wie Diäthylenglykoldimethyläther durchgeführt werden. Die Reaktion kann durch Hitzeanwendung, z.B. durch Erhitzen zum Siedepunkt des Lösungsmittels, beschleunigt werden.

Verbindungen der Formel I, in welcher $R^5$ oder $R^6$ einen Acylrest bedeuten, können aus Verbindungen der Formel I, in welcher wenigstens einer der Reste $R^5$ oder $R^6$ Wasserstoff bedeutet, durch Behandlung mit einem Acylhalogenid oder Acylanhydrid hergestellt werden, wobei die Acylgruppe eine Alkanoylgruppe mit höchstens 6 Kohlenwasserstoffatomen, z.B. die Acetylgruppe, oder die Benzoylgruppe ist, in welcher der Phenylkern wie oben beschrieben substituiert sein kann und wobei das Halogenid z.B. das Chlorid sein kann. Die Reaktion kann in Gegenwart einer Base, wie z.B. einem Alkalicarbonat, z.B. Kaliumcarbonat, oder einem tertiären Amin z.B. Triäthylamin, durchgeführt werden. Durch Erhitzen zum Siedepunkt des Acylierungsmittels kann die Reaktion beschleunigt werden.

Die als Zwischenprodukte verwendeten Barbitursäurederivate der Formel III können nach literaturbekannten Methoden hergestellt werden. Wenn z.B. $R^4$ Wasserstoff und X Sauerstoff bedeuten, kann man entsprechend dem folgenden Reaktionsschema

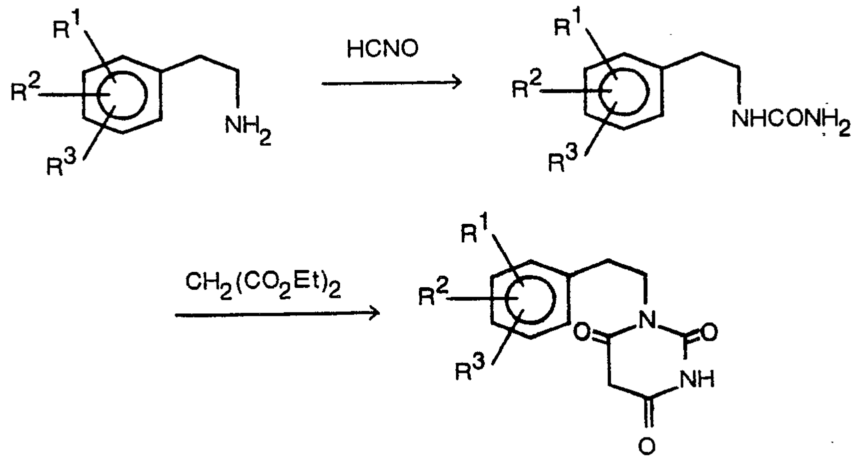


nach dem von C. F. Kurzer in Organic Synthesis, Coll. Vol. 4 (1963) beschriebenen Verfahren zur Harnstoffbildung und dem von J. B. Dickey und A. R. Gray in Organic Synthesis, Coll. Vol. 2 (1943) beschriebenen Verfahren zur Herstellung von Barbitursäuren verfahren.

Die Pyrimido-(6,1-a)-isochinolin-4-on-derivate gemäß der Formel I besitzen wertvolle pharmakodynamische Eigenschaften, z.B. blutdrucksenkende, bronchodilatierende und antiallergische Wirksamkeit.

Aufgrund der blutdrucksenkenden Wirkung sind die neuen Wirkstoffe für die Behandlung und Prophylaxe von Herz-Kreiskauferkrankungen wie z.B. essentielle und maligne Hypertonie, Herzinsuffizienz, Angina pectoris und Störungen des peripheren Kraislaufs geeignet. Die Wirkstoffe können auch in Verbindung mit anderen pharmakologisch wirksamen Substanzen eingesetzt werden z.B. mit Diuretika, Antiarrhythmika, ß-Blockern, Beruhigungsmitteln, herzgefäßerweiternden Mitteln, Hypolipidemika usw.

Aufgrund der bronchodilatierenden und antiallergischen Wirkung sind die neuen Wirkstoffe für die Behandlung und Prophylaxe von Erkrankungen der Atemwege wie z.B. Bronchialasthma, chronische Bronchitis und Emphysem und Allergien wie z.B. allergisches Asthma, Heufieber, allergische Rhinitis, Konjunktivitis, Urticaria usw. geeignet. Die Wirkstoffe können auch in Verbindung mit anderen pharmakologisch wirksamen Substanzen eingesetzt werden, wie z.B. Corticosteroiden, Sympathomimetika, Xanthinderivaten, Antihistaminika, Beruhigungsmitteln, Herzmittel usw.

Die erfindungsgemäßen Wirkstoffe können peroral, parenteral (intramuskulär, intravenös, subkutan), rektal, als Aerosol verabreicht oder topisch angewandt werden.

Beispiel 1

9,10-Dimethoxy-3,4,6,7-tetrahydro-2H-pyrimido-(6,1-a)-isochinolin-2,4-dion (Formel II)

10 g Polyphosphorsäure werden auf 105°C erhitzt und 1,0 g 1-(3,4-Dimethoxyphenyläthyl)-barbitursäure wird unter Rühren zugesetzt. Das Reaktionsgemisch wird 4 Stunden lang erhitzt und dann auf zerkleinertes Eis gegossen, wobei ein weißer Feststoff entsteht, der abfiltriert, mit Wasser gewaschen, getrocknet und aus Dimethylamid umkristallisiert wird.
Ausbeute: 500 mg, Schmelzpunkt: 323—325°C

Beispiel 2

2-Chlor-9,10-dimethoxy-6,7-dihydro-2H-pyrimido-(6,1-a)-isochinolin-4-on (Formel II)

Eine Mischung aus 20,2 g 1-(3,4-Dimethoxyphenyläthyl)barbitursäure und 100 ml Phosphoroxychlorid wird 2,5 Stunden bei einer Temperatur von 100 bis 110°C unter Rückfluß erhitzt. Überschüssiges Phosphoroxychlorid wird abdestilliert. Der Rückstand wird auf zerkleinertes Eis gegossen und mit einer kalten wässrigen 30 %igen Natriumhydroxidlösung basisch gemacht. Der dabei gebildete gelbe, gummiartige Niederschlag wird abgetrennt und mit Chloroform extrahiert. Der Extrakt wird mit Wasser gewaschen, über Natriumsulfat getrocknet und unter vermindertem Druck zur Trockne eingedampft. Der Rückstand wird an einer Silicagelkolonne mit Chloroform als Eluiermittel zur gewünschten Verbindung gereinigt.
Ausbeute: 20,2 g (ca. 100%), Schmelzpunkt: 235—236°C.

Beispiel 3

2-Chlor-8,10-methylendioxy-6,7-dihydro-2H-pyrimido-(6,1-a)-isochinolin-4-on (Formel II)

Es wird analog Beispiel 2 verfahren, wobei statt 1-(3,4-Dimethoxyphenyläthyl)-barbitursäure 1-(3,4-Methylendioxyphenyläthyl)-barbitursäure eingesetzt wird.
Ausbeute: 80%, Schmelzpunkt: 245—247°C.

Beispiel 4

9,10-Dimethoxy-3-methyl-3,4,5,7-tetrahydro-2H-pyrimido-(6,1-a)-isochinolin-2,4-dion (Formel II)

Ein Gemisch aus 9,10 Dimethoxy-3,4,6,7-tetrahydro-2H-pyrimido-(6,1-a)-isochinolin-2,4-dion (4,11 g), ölfreiem Natriumhydrid (0,75 g) und Dimethylformamid (100 ml) wird 15 Minuten auf 100°C erhitzt und dann auf Raumtemperatur abgekühlt. Hierzu wird Methyliodid (10 ml) gegeben. Das Reaktionsgemisch wird 12 Stunden auf 100°C erhitzt. Das Lösungsmittel wird im Vakuum entfernt und der Rückstand mit kaltem Wasser behandelt. Der Feststoff wird abfiltriert und aus Essigester-Methylenchlorid umkristallisiert. Ausbeute: 4,0 g, Schmelzpunkt: 260—262°C.

Beispiel 5

9,10-Dimethoxy-2-tert.-butylamino-6,7-dihydro-4H-pyrimido-(6,1-a)-isochinolin-4-on-hydrochlorid (Formel I)

Eine Lösung aus 9,10-Dimethoxy-2-chlor-6,7-dihydro-4H-pyrimido-(6,1-a)-isochinolin-4-on (3,0 g) und tert.-Butyl-amin (10,0 ml) in Chloroform (75,0 ml) wird 16 Stunden lang unter Rückfluß erhitzt. Das Lösungsmittel wird bei vermindertem Druck verdampft und der Rückstand mit einer verdünnten Natriumhydroxidlösung zu einem weißen Niederschlag verrieben. Der Niederschlag wird abfiltriert, getrocknet, in Äthanol gelöst und durch Behandlung mit Salzsäure in sein Hydrochlorid übergeführt. Das Hydrochlorid wird aus einem Äthanol/Äther-Gemisch kristallisiert.
Ausbeute: 3,0 g, Schmelzpunkt: 265—270°C.

**Patentansprüche für die Vertragsstaaten: BE CH DE FR GB IT NL SE**

1. Verbindungen der Formel I

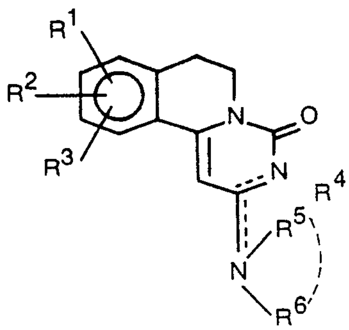

in welcher

$R^1$ und $R^2$ unabhängig voneinander Wasserstoff, Alkoxy mit 1 bis 3 Kohlenstoffatomen, Hydroxy-, Methylendioxy oder Äthylendioxy bedeuten,

$R^3$ Wasserstoff oder Alkoxy mit 1 bis 3 Kohlenstoffatomen bedeutet,

$R^4$ Wasserstoff oder Alkyl mit 1 bis 4 Kohlenstoffatomen, oder Di($C_{1-4}$-Alkyl)-amino-$C_{1-4}$-alkyl) bedeutet und $R^4$ für ein Elektronenpaar steht, falls $R^5$ für einen der unten angegebenen Reste steht,

$R^5$ Wasserstoff, Alkyl mit 1 bis 6 Kohlenstoffatomen, Hydroxyalkyl mit 1 bis 6 Kohlenstoffatomen, Alkanoyl mit 1 bis 6 Kohlenstoffatomen oder Benzoyl, wobei der Phenylrest 1—3-fach substituiert sein kann mit Halogen, Trifluormethyl, $C_1$—$C_3$-Alkyl, $C_1$—$C_3$-Alkoxy, Amino, Hydroxy oder O-Alkali, bedeutet und $R^5$ für ein Elektronenpaar steht, falls $R^4$ für einen der oben angegebenen Reste steht,

$R^6$ Wasserstoff, Hydroxyl, Amino, $C_1$—$C_3$-Alkylamino, Di-$C_1$—$C_3$-Alkylamino, $C_1$—$C_6$-Alkyl, das substituiert sein kann mit Halogen, Hydroxy, $C_1$—$C_3$-Alkoxy, Amino oder Di-($C_1$—$C_4$-alkyl)-amino, Cyclohexyl Phen-$C_1$—$C_2$-alkyl, Phenyl, welches 1—3-fach substituiert sein kann mit Halogen. Trifluormethyl, $C_1$—$C_3$-Alkyl, $C_1$—$C_3$-Alkoxy, Amino, Hydroxy oder O-Alkali, oder $R^5$ und $R^6$ zusammen mit dem N-Atom, an das sie gebunden sind, den Pyrrolidino- oder Morpholino-Rest oder den Piperazino-Rest, welche, am N durch $C_1$—$C_3$-Alkyl, $C_2$—$C_4$-Alkoxycarbonyl oder Phenyl, welches 1—3 fach substituiert sein kann mit Halogen, Trifluormethyl, $C_1$—$C_3$-Alkyl, $C_1$—$C_3$-Alkoxy, Amino, Hydroxy oder O-Alkali, substituiert sein können, sowie deren Säureadditionssalze und quaternären Ammoniumsalze.

2. Verbindungen der Formel II

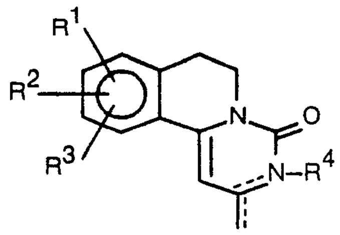

II

worin $R^1$, $R^2$ und $R^3$ die zur Formel I gegannte Bedeutung haben, jedoch zusätzlich Halogen darstellen können, $R^4$ die Bedeutung der zur Formel I genannten Substituenten hat, in welchem Falle X Schwefel bedeutet, oder ein Elektronenpaar darstellt, in welchem Falle X ein Halogenatom darstellt.

3. Verfahren zur Herstellung einer Verbindung der Formel II

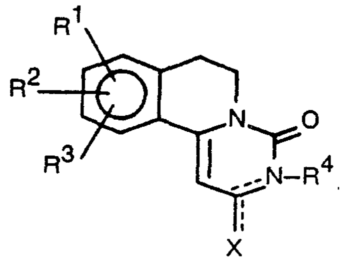

II

worin $R^1$, $R^2$, $R^3$, $R^4$ und X die in Anspruch 2 genannten Bedeutungen haben, dadurch gekennzeichnet, daß ein Barbitursäurederivat der Formel III

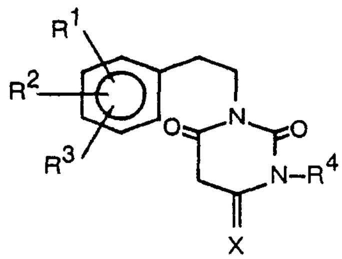

III

worin $R^1$, $R^2$, $R^3$ und $R^4$ die zur Formel I genannten Bedeutungen haben und X Schwefel bedeutet, einer Kondensationsreaktion unterzogen wird entweder in Gegenwart einer Säure, wobei eine Verbindung der Formel II gebildet wird, worin X die oben genannten Bedeutungen hat, oder in Gegenwart eines anorganischen Säurehalogenids oder von Phosphorpentahalogenid, wobei eine Verbindung der Formel II gebildet wird, worin X ein Halogenatom darstellt.

4. Verfahren nach Anspruch 3, dadurch gekennzeichnet, daß eine Verbindung der Formel III mit Phosphorsäure bei einer Temperatur von 80—150°C umgesetzt wird.

5. Verfahren nach Anspruch 3, dadurch gekennzeichnet, daß eine Verbindung der Formel III in Gegenwart von Phosphoroxychlorid, Phosphorpentachlorid oder Thionylchlorid und eines Lösungsmittels umgesetzt wird.

6. Verfahren zur Herstellung einer Verbindung der Formel I

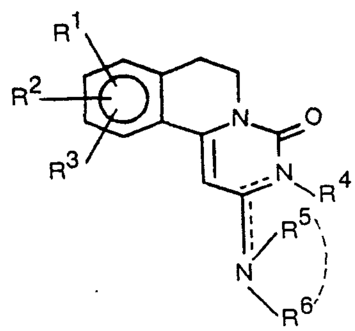

worin $R^1$, $R^2$, $R^3$, $R^4$, $R^5$ und $R^6$ die in Anspruch 1 genannten Bedeutungen haben, dadurch gekennzeichnet, daß eine Verbindung der Formel II

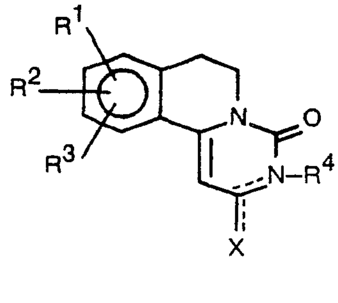

II

worin $R^1$, $R^2$, $R^3$, $R^4$ und X die in Anspruch 2 genannten Bedeutungen haben, mit einer Verbindung der Formel

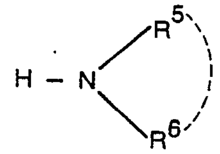

worin $R^5$ und $R^6$ die zur Formel I in Anspruch 1 genannten Bedeutungen haben, in Gegenwart einer Base umgesetzt wird.

7. Arzneimittel, gekennzeichnet durch den Gehalt an einer Verbindung der Formel I und einem pharmazeutisch üblichen Träger und/oder Stabilisator.

8. Verbindungen der Formel I zur Verwendung als Arzneimittel.

**Patentansprüche für den Vertragsstaat: AT**

1. Verfahren zur Herstellung einer Verbindung der Formel II

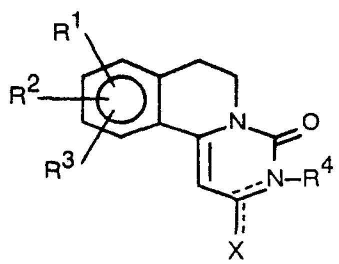

II

in welcher

$R^1$ und $R^2$ unabhängig voneinander Wasserstoff, Alkoxy mit 1 bis 3 Kohlenstoffatomen, Hydroxy-, Methylendioxy oder Äthylendioxy bedeuten,

$R^3$ Wasserstoff oder Alkoxy mit 1 bis 3 Kohlenstoffatomen bedeutet,

$R^4$ Wasserstoff oder Alkyl mit 1 bis 4 Kohlenstoffatomen, oder Di($C_{1-4}$-Alkyl)-amino-$C_{1-4}$-alkyl) bedeutet, in welchem Falle X Schwefel darstellt, oder $R^4$ ein Elektronenpaar bedeutet, in welchem Falle X ein Halogenatom darstellt, dadurch gekennzeichnet, daß eine Barbitursäurederivat der Formel III

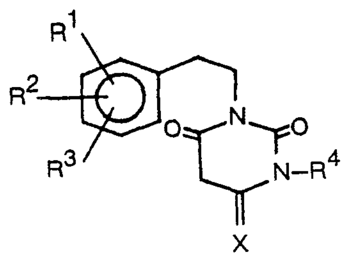

III

worin $R^1$, $R^2$, $R^3$ und $R^4$ die zur Formel II genannten Bedeutungen haben und X Schwefel bedeutet, einer Kondensationsreaktion unterzogen wird entweder in Gegenwart einer Säure, wobei eine Verbindung der Formel II gebildet wird, worin X die oben genannten Bedeutungen hat, oder in Gegenwart eines anorganischen Säurehalogenids oder von Phosphorpentahalogenid, wobei eine Verbindung der Formel II gebildet wird, worin X ein Halogenatom darstellt.

2. Verfahren nach Anspruch 1, dadurch gekennzeichnet, daß eine Verbindung der Formel III mit Phosphorsäure bei einer Temperatur von 80—150°C umgesetzt wird.

3. Verfahren nach Anspruch 1, dadurch gekennzeichnet, daß eine Verbindung der Formel III in Gegenwart von Phosphoroxychlorid, Phosphorpentachlorid oder Thionylchlorid und eines Lösungsmittels umgesetzt wird.

4. Verfahren zur Herstellung einer Verbindung der Formel I

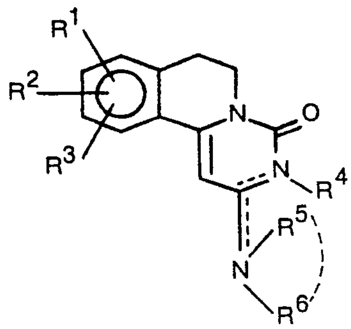

worin $R^1$, $R^2$, $R^3$ und $R^4$ die in Anspruch 1 genannten Bedeutungen haben

$R^5$ Wasserstoff, Alkyl mit 1 bis 6 Kohlenstoffatomen, Hydroxyalkyl mit 1 bis 6 Kohlenstoffatomen, Alkanoyl mit 1 bis 6 Kohlenstoffatomen oder Benzoyl, wobei der Phenylrest 1—3-fach substituiert sein kann mit Halogen, Trifluormethyl, $C_1$—$C_3$-Alkyl, $C_1$—$C_3$-Alkoxy, Amino, Hydroxy oder O-Alkali, bedeutet und $R^5$ für ein Elektronenpaar steht, falls $R^4$ für einen der oben angegebenen Reste steht,

$R^6$ Wasserstoff, Hydroxyl, Amino, $C_1$—$C_3$-Alkylamino, Di-$C_1$—$C_3$-Alkylamino, $C_1$—$C_6$-Alkyl, das substituiert sein kann mit Halogen, Hydroxy, $C_1$—$C_3$-Alkoxy, Amino oder Di-($C_1$—$C_4$-alkyl)-amino, Cyclohexyl Phen-$C_1$—$C_2$-alkyl, Phenyl, welches 1—3-fach substituiert sein kann mit Halogen, Trifluormethyl, $C_1$—$C_3$-Alkyl, $C_1$—$C_3$-Alkoxy, Amino, Hydroxy oder O-Alkali, oder $R^5$ und $R^6$ zusammen mit dem N-Atom, an das sie gebunden sind, den Pyrrolidino- oder Morpholino-Rest oder den Piperazino-Rest, welche, am N durch $C_1$—$C_3$-Alkyl, $C_2$—$C_4$-Alkoxycarbonyl oder Phenyl, welches 1—3 fach substituiert sein kann mit Halogen, Trifluormethyl, $C_1$—$C_3$-Alkyl, $C_1$—$C_3$-Alkoxy, Amino, Hydroxy oder O-Alkali, substituiert sein können, sowie deren Säureadditionssalze und quaternären Ammoniumsalze, dadurch gekennzeichnet, daß eine Verbindung der Formel II

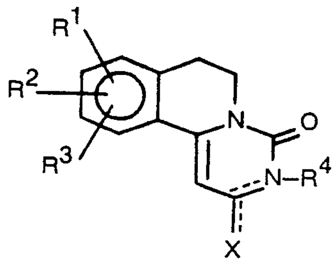

II

worin $R^1$, $R^2$, $R^3$, $R^4$ und X die in Anspruch 1 genannten Bedeutungen haben, mit einer Verbindung der Formel

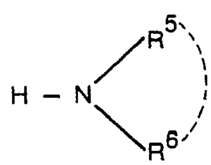

worin $R^5$ und $R^6$ die zur Formel I genannten Bedeutungen haben, in Gegenwart einer Base umgesetzt wird.

**Revendications pour les Etats contractants: BE CH DE FR GB IT NL SE**

1. Composés répondant à la formule I

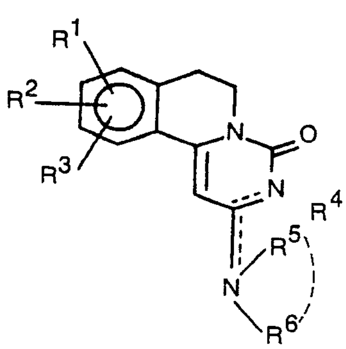

dans laquelle

$R^1$ et $R^2$ désignent indépendamment l'un de l'autre l'hydrogène, un groupe alcoxy en C1 à C3, un groupe hydroxy, méthylènedioxy ou éthylènedioxy,

$R^3$ désigne l'hydrogène ou un groupe alcoxy en C1 à C3,

$R^4$ désigne l'hydrogène ou un groupe alkyle en C1 à C4, ou un groupe di(alkyle en C1 à C4)-amino-(alkyle en C1 à C4) et $R^4$ désigne une paire d'électrons au cas où $R^5$ représente un des radicaux indiqués ci-dessous,

$R^5$ désigne l'hydrogène, un groupe alkyle en C1 à C6, hydroxyalkyle en C1 à C6, alcanoyle en C1 à C6, ou benzoyle dans lequel le radical phényle peut être substitué une à trois fois par un halogène, un groupe trifluorométhyle, alkyle en C1 à C3, alcoxy en C1 à C3, amino, hydroxy ou O-métal alcalin, et $R^5$ représente une paire d'électrons au cas où $R^4$ représente un des radicaux indiqués ci-dessus;

$R^6$ désigne l'hydrogène, un groupe hydroxyle, amino, alkyle en C1 à C3) amino, di(alkyle en C1 à C3) amino, alkyle en C1 à C6, qui peut être substitué par un halogène, un groupe hydroxy, alcoxy en C1 à C3, amino ou di(alkyle en C1 à C4) amino, cyclohexyle, phényl-(alkyle en C1 ou C2), phényle pouvant être substitué une à trois fois par un halogène, trifluorométhyle, alkyle en C2 à C3, alcoxy en C1 à C3, amino, hydroxy ou O-métal alcalin, ou bien $R^5$ et $R^6$ forment avec l'atome de N auquel ils sont rattachés un radical pyrrolidino ou morpholino, ou le radical pipérazino, pouvant être substitués sur le N par un groupe alkyle en C1 à C3, (alcoxy en C2 à C4)-carbonyle ou phényle qui peut lui-même être substitué une à trois fois par un halogène, un groupe trifluorométhyle, alkyle en C1 à C3, alcoxy en C1 à C3, amino, hydroxy ou O-métal alcalin, ainsi que leurs sels d'addition aux acides et leurs sels d'ammonium quaternaire.

2. Composés répondant à la formule II

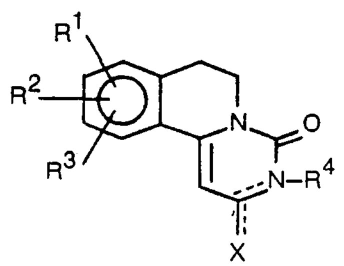

II

dans laquelle

$R^1$, $R^2$ et $R^3$ ont la signification indiquée à propos de la formule I, mais peuvent représenter en outre un halogène, $R^4$ représente les substituants indiqués à propos de la formule I, auquel cas X désigne le soufre, ou représente une paire d'électrons, auquel cas X représente un atome d'halogène.

3. Procédé de préparation d'un composé répondant à la formule II

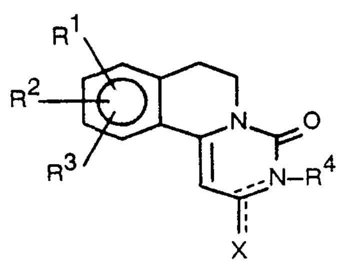

II

dans laquelle

$R^1$, $R^2$, $R^3$, $R^4$ et X ont les significations indiquées dans la revendication 2, caractérisé en ce qu'on soumet à une réaction de condensation un dérivé de l'acide barbiturique répondant à la formule III

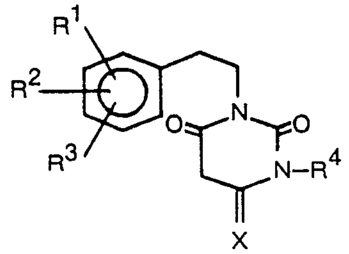

III

dans laquelle

$R^1$, $R^2$, $R^3$ et $R^4$ ont les significations indiquées à propos de la formule I et X désigne le soufre, soit en présence d'un acide, auquel cas il se forme ou composé répondant à la formule II, dans laquelle X a les significations indiquées ci-dessus, soit en présence d'un halogénure d'acide minéral ou d'un penta-halogénure de phosphore, auquel cas il se forme un composé répondant à la formule II, dans laquelle X représente un atome d'halogène.

4. Procédé suivant la revendication 3, caractérisé en ce qu'on fait réagir un composé répondant à la formule III avec de l'acide phosphorique à une température de 80 à 150°C.

5. Procédé suivant la revendication 3, caractérisé en ce qu'on fait réagir un composé répondant à la formule III en présence d'oxychlorure de phosphore, de pentachlorure de phosphore ou de chlorure de thionyle et d'un solvant.

6. Procédé de préparation d'un composé répondant à la formule I

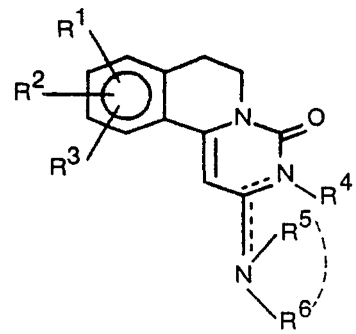

dans laquelle

$R^1$, $R^2$, $R^3$, $R^4$, $R^5$ et $R^6$ ont les significations indiquées dans la revendication 1, caractérisé en ce qu'on fait réagir un composé répondant à la formule II

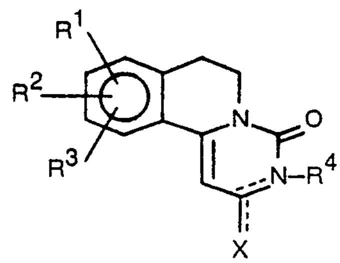

II

dans laquelle

$R^1$, $R^2$, $R^3$, $R^4$ et X ont les significations indiquées dans la revendication 2, avec un composé répondant à la formule

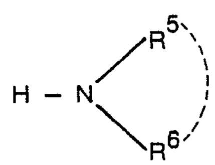

dans laquelle

$R^5$ et $R^6$ ont les significations indiquées à propos de la formule I dans la revendication 1, en présence d'une base.

7. Médicament, caractérisé en ce qu'il contient un composé répondant à la formule I et un excipient et/ou stabilisant habituel en pharmacie.

8. Composés répondant à la formule I pour l'utilisation comme médicaments.

**Revendications pour l'Etat contractant: AT**

1. Procédé de préparation d'un composé répondant à la formule II

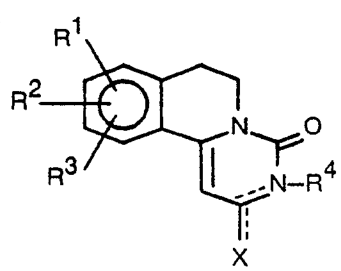

II

dans laquelle

$R^1$ et $R^2$ désigent indépendamment l'un de l'autre l'hydrogène, un groupe alcoxy en C1 à C3, un groupe hydroxy, méthylènedioxy ou éthylènedioxy,

$R^3$ désigne l'hydrogène ou un groupe alcoxy en C1 à C3,

R⁴ désigne l'hydrogène ou un groupe alkyle en C1 à C4, ou un groupe di(alkyle en C1 à C4)-amino-(alkyle en C1 à C4), auquel cas X représente le soufre, ou bien $R^4$ désigne une paire d'électrons auquel cas X représente un atome d'halogène, caractérisé en ce qu'on soumet à une réaction de condensation un dérivé de l'acide barbiturique répondant à la formule III

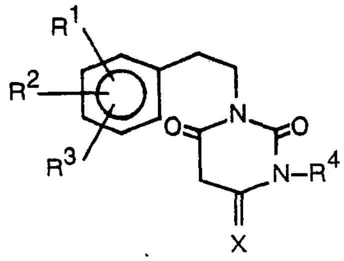

III

dans laquelle

$R^1$, $R^2$, $R^3$ et $R^4$ ont les significations indiquées à propos de la formule II, et X désigne le soufre, soit en présence d'un acide, auquel cas il se forme ou composé répondant à la formule II, dans laquelle X a les significations indiquées ci-dessus, soit en présence d'un halogénure d'acide minéral ou d'un pentahalogénure de phosphore, auquel cas il se forme un composé répondant à la formule II, dans laquelle X désigne un atome d'halogène.

2. Procédé suivant la revendication 1, caractérisé en ce qu'on fait réagir un composé répondant à la formule III avec de l'acide phosphorique à une température de 80—150°C.

3. Procédé suivant la revendication 1, caractérisé en ce qu'on fait réagir un composé répondant à la formule III en présence d'oxychlorure de phosphore, de pentachlorure de phosphore ou de chlorure de thionyle et d'un solvant.

4. Procédé de préparation d'un composé répondant à la formule I

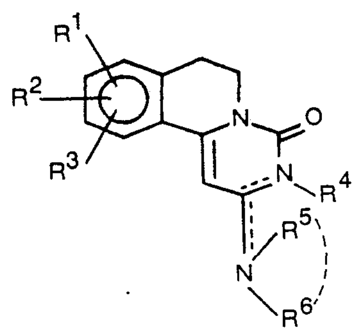

I

dans laquelle

$R^1$, $R^2$, $R^3$ et $R^4$ ont les significations indiquées dans la revendication 1, et

$R^5$ désigne l'hydrogène, un groupe alkyle en C1 à C6, un groupe hydroxyalkyle en C1 à C6, un groupe alcanoyle en C1 à C6 ou un groupe benzoyle, dans lequel le radical phényle peut être substitué une à trois fois par un halogène, un groupe trifluorométhyle, alkyle en C1 à C3, alcoxy en C1 à C3, amino, hydroxy ou O-métal alcalin, et $R^5$ désigne une paire d'électrons au cas où $R^4$ représente un des radicaux indiqués ci-dessus;

$R^6$ désigne l'hydrogène, un groupe hydroxyle, amino, (alkyle en C1 à C3)amino, di(alkyle en C1 à C3)amino, alkyle en C1 à C6 pouvant être substitué par un halogène, un groupe hydroxy, un groupe alcoxy en C1 à C3, amino ou di (alkyle en C1 à C4)-amino, cyclohexyle, phényl-(alkyle en C1 ou C2), phényle pouvant être substitué une à trois fois par un halogène, un groupe trifluorométhyle, (alkyle en C1 à C3), alcoxy en C1 à C3, amino, hydroxy ou O-métal alcalin ou bien $R^5$ et $R^6$ forment avec l'atome de N auquel ils sont rattachés un radical pyrrolidino ou morpholino, ou le radical pipérazino, pouvant être substitués sur le N par un groupe alkyle en C1 à C3, (alcoxy en C2 à C4)-carbonyle ou phényle qui peut être substitué une à trois fois par un halogène, un groupe trifluorométhyle, alkyle en C1 à C3, alcoxy en C1 à C3, amino, hydroxy ou O-métal alcalin, ainsi que leurs sels d'addition aux acides et leurs sels d'ammonium quaternaire, caractérisé en ce qu'on fait réagir un composé répondant à la formule II

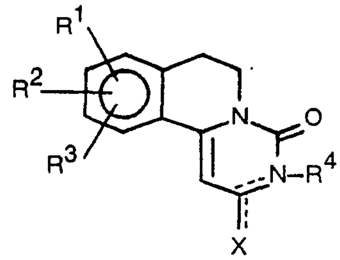

II

dans laquelle

$R^1$, $R^2$, $R^3$, $R^4$ et X ont les significations indiquées dans la revendication 1, avec un composé répondant à la formule

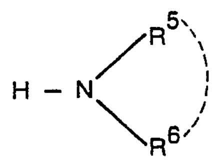

dans laquelle

$R^5$ et $R^6$ ont les significations indiquées à propos de la formule I, en présence d'une base.

**Claims for the Contracting States: BE CH DE FR GB IT NL SE**

1. Compounds of the formula I

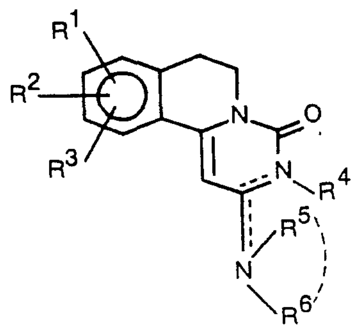

I

in which

$R^1$ and $R^2$ independently stand for hydrogen, hydroxy or $C_1$—$C_3$-alkoxy, or taken together, form a methylenedioxy or an ethylenedioxy group,

$R^3$ stands for hydrogen or $C_1$—$C_3$-alkoxy,

$R^4$ stands for hydrogen, $C_1$—$C_4$-alkyl or di($C_1$—$C_4$-alkyl)-amino-$C_1$—$C_4$-alkyl or stands for an electron pair, if $R_5$ stands for one of the following radicals,

$R^5$ stands for hydrogen, $C_1$—$C_6$-alkyl, $C_1$—$C_6$-hydroxyalkyl, $C_1$—$C_6$-alkanoyl or benzoyl wherein the phenyl moiety may be substituted 1 to 3 times with halogen, trifluoromethyl, $C_1$—$C_3$-alkyl, $C_1$—$C_3$-alkoxy, amino, hydroxy or O-alkalimetal, or $R^5$ stands for an electron pair if $R^4$ stands for one of the above radicals,

$R^6$ stands for hydrogen, hydroxy, amino, $C_1$—$C_3$-alkylamino, di-($C_1$—$C_3$-alkyl)-amino, $C_1$—$C_6$-alkyl which may be substituted with halogen, hydroxy, $C_1$—$C_3$-alkoxy, amino or di-($C_1$—$C_4$-alkyl)-amino, cyclohexyl, phenyl-$C_1$—$C_2$-alkyl, phenyl, which may be substituted 1 to 3 times with halogen, trifluoromethyl, $C_1$—$C_3$-alkyl, $C_1$—$C_3$-alkoxy, amino, hydroxy or O-alkalimetal, or

$R^5$ and $R^6$ when taken together with the N-atom to which they are bound, stand for the pyrrolidino- or morpholino radical or the piperazino radical which may be substituted at the N-atom with $C_1$—$C_3$-alkyl, $C_2$—$C_4$-alkoxycarbonyl or phenyl which in turn may be substituted 1 to 3 times with halogen, trifluoromethyl, $C_1$—$C_3$-alkyl, $C_1$—$C_3$-alkoxy, amino, hydroxy or O-alkalimetal, and the acid addition salts and quaternary ammonium salts thereof.

2. A compound of the formula II

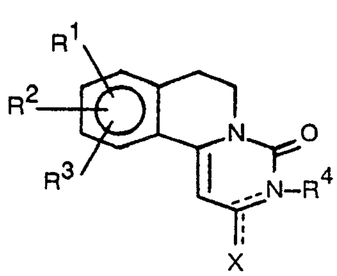

II

wherein

$R^1$, $R^2$ and $R^3$ are as defined in formula I above and additionally stand for halogen, and $R^4$, represents the substituents defined in formula I above, in which case X stands for sulfur, or $R^4$ represents an electron pair, in which case X is a halogen atom.

3. Process for the manufacture of a compound of the formula II

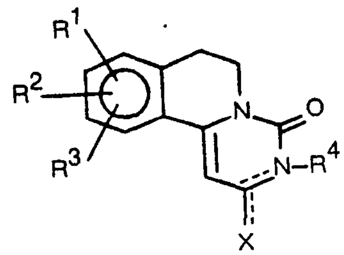

II

in which

$R^1$, $R^2$, $R^3$, $R^4$ and X are as defined in claim 2, which comprises subjecting a barbituric acid derivative of the formula III

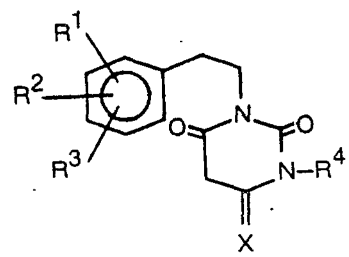

III

wherein

$R^1$, $R^2$, $R^3$ and $R^4$ are as defined in formula I and X stands for sulfur, to a condensation reaction either in the presence of an acid to yield a compound of the formula II, wherein X is as defined above, or in the presence of an inorganic acid halide or phosphorus pentahalide, to yield a compound of the formula II, wherein X stands for a halogen atom.

4. The process of claim 3, wherein a compound of the formula III is reacted with phosphoric acid at a temperature of from 80 to 150°C.

5. The process of claim 3, wherein a compound of the formula III is reacted in the presence of a phosphorus oxychloride, phosphorus pentachloride or thionyl chloride and in the presence of a solvent.

6. Process for the manufacture of a compound of the formula I

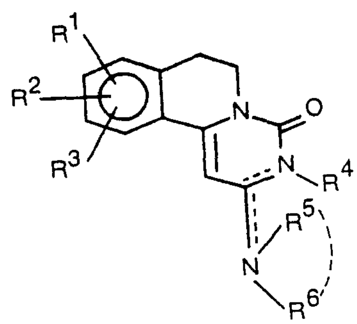

I

in which

$R^1$, $R^2$, $R^3$, $R^4$, $R^5$ and $R^6$ are as defined in claim 1, which comprises reacting a compound of the formula II

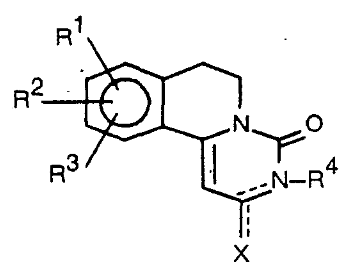

II

in which

$R^1$, $R^2$, $R^3$, $R^4$ and X are as defined in claim 2, with a compound of the formula

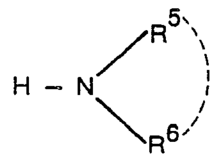

in which

$R^5$ and $R^6$ are as defined in claim 1, in the presence of a base.

7. Medicament containing a compound of the formula I and a pharmaceutically acceptable carrier and/or stabilizer.

8. Compounds of the formula I for use as medicaments.

**Claims for the Contracting State: AT**

1. Process for the manufacture of a compound of the formula II

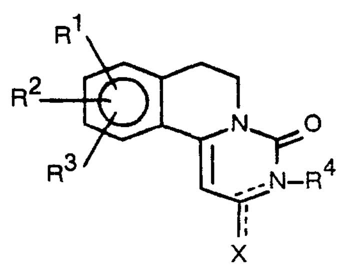

II

in which

$R^1$ and $R^2$ independently stand for hydrogen, $C_1$—$C_3$-alkoxy, or hydroxy or taken together form a methylenedioxy or an ethylenedioxy group,

$R^3$ stands for hydrogen or $C_1$—$C_3$-alkoxy,

$R^4$ stands for hydrogen, $C_1$—$C_4$-alkyl or di($C_1$—$C_4$-alkyl)-amino-$C_1$—$C_4$-alkyl, in which case X represents sulfur, or

$R^4$ stands for an electron pair, in which case X represents halogen, which comprises subjecting a barbituric acid derivative of the formula III

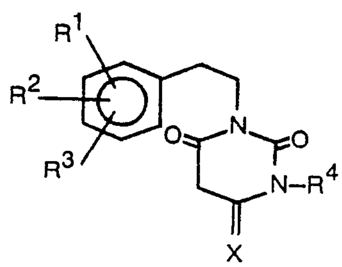

III

wherein

$R^1$, $R^2$, $R^3$ and $R^4$ are as defined in formula I and X stands for sulfur, to a condensation reaction either in the presence of an acid to yield a compound of the formula II, wherein X is as defined above, or in the presence of an inorganic acid halide or phosphorus pentahalide, to yield a compound of the formula II, wherein X stands for a halogen atom.

2. The process of claim 1, wherein a compound of the formula III is reacted with phosphoric acid at a temperature of from 80 to 150°C.

3. The process of claim 3, wherein a compound of the formula III is reacted in the presence of a phosphorus oxychloride, phosphorus pentachloride or thionyl chloride and in the presence of a solvent.

4. Process for the manufacture of a compound of the formula I

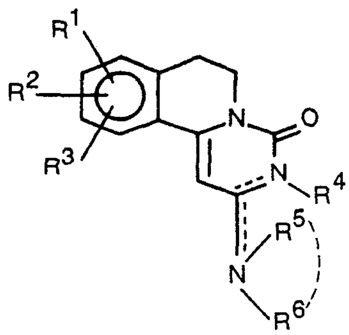

in which

$R^1$ and $R^2$ independently stand for hydrogen, hydroxy or $C_1$—$C_3$-alkoxy, or taken together form a methylenedioxy or an ethylenedioxy group,

$R^3$ stands for hydrogen or $C_1$—$C_3$-alkoxy,

$R^4$ stands for hydrogen, $C_1$—$C_4$-alkyl or di($C_1$—$C_4$-alkyl)-amino-$C_1$—$C_4$-alkyl or stands for an electron pair, if $R_5$ stands for one of the following radicals

$R^5$ stands for hydrogen, $C_1$—$C_6$-alkyl, $C_1$—$C_6$-hydroxyalkyl, $C_1$—$C_6$-alkanoyl or benzoyl wherein the phenyl moiety may be substituted 1 to 3 times with halogen, trifluoromethyl, $C_1$—$C_3$-alkyl, $C_1$—$C_3$-alkoxy, amino, hydroxy or O-alkalimetal, or $R^5$ stands for an electron pair if $R^4$ stands for one of the above radicals,

$R^6$ stands for hydrogen, hydroxy, amino, $C_1$—$C_3$-alkylamino, di-($C_1$—$C_3$-alkyl)-amino, $C_1$—$C_6$-alkyl which may be substituted with halogen, hydroxy, $C_1$—$C_3$-alkoxy, amino or di-($C_1$—$C_4$-alkyl)-amino, cyclohexyl, phenyl-$C_1$—$C_2$-alkyl, phenyl, which may be substituted 1 to 3 times with halogen, trifluoromethyl, $C_1$—$C_3$-alkyl, $C_1$—$C_3$-alkoxy, amino, hydroxy or O-alkalimetal, or

$R^5$ and $R^6$ when taken together with the N-atom to which they are bound, stand for the pyrrolidino- or morpholino radical or the piperazino radical which may be substituted at the N-atom with $C_1$—$C_3$-alkyl, $C_2$—$C_4$-alkoxycarbonyl or phenyl which in turn may be substituted 1 to 3 times with halogen, trifluoromethyl, $C_1$—$C_3$-alkyl, $C_1$—$C_3$-alkoxy, amino, hydroxy or O-alkalimetal, and the acid addition salts and quaternary ammonium salts thereof.